(19)

**Europäisches Patentamt**
**European Patent Office**
**Office européen des brevets**

(11) **EP 4 362 811 B1**

(12) **EUROPEAN PATENT SPECIFICATION**

(45) Date of publication and mention
of the grant of the patent:
**25.02.2026  Bulletin 2026/09**

(21) Application number: **22747491.3**

(22) Date of filing: **28.06.2022**

(51) International Patent Classification (IPC):
***A61B 8/08*** *(2006.01)*       ***A61B 8/00*** *(2006.01)*

(52) Cooperative Patent Classification (CPC):
**A61B 8/085; A61B 8/0858; A61B 8/4488;**
**A61B 8/485; A61B 8/5207; A61B 8/5223**

(86) International application number:
**PCT/US2022/035315**

(87) International publication number:
**WO 2023/278445 (05.01.2023 Gazette 2023/01)**

(54) **SYSTEM AND METHOD FOR NON-INVASIVE DETERMINATION OF PRESSURE IN A BIOLOGICAL COMPARTMENT**

SYSTEM UND VERFAHREN ZUR NICHT-INVASIVEN BESTIMMUNG DES DRUCKS IN EINEM
BIOLOGISCHEN KOMPARTIMENT

SYSTÈME ET MÉTHODE POUR LA DÉTERMINATION NON INVASIVE DE LA PRESSION DANS UN
COMPARTIMENT BIOLOGIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.06.2021  US 202163215912 P**

(43) Date of publication of application:
**08.05.2024  Bulletin 2024/19**

(73) Proprietor: **Mayo Foundation for Medical**
**Education and Research**
**Rochester, Minnesota 55905 (US)**

(72) Inventors:
 • **FATEMI, Mostafa**
  **Rochester, Minnesota 55902-4507 (US)**
 • **ALIZAD, Azra**
  **Rochester, Minnesota 55902-4507 (US)**
 • **ROSEN, David P.**
  **Rochester, Minnesota 55901 (US)**

(74) Representative: **Samson & Partner Patentanwälte**
**mbB**
**Widenmayerstraße 6**
**80538 München (DE)**

(56) References cited:
**US-A1- 2014 296 709**

• **WIDMAN ERIK ET AL: "Feasibility of shear wave elastography for plaque characterization", 2014 IEEE INTERNATIONAL ULTRASONICS SYMPOSIUM, IEEE, 3 September 2014 (2014-09-03), pages 1818 - 1821, XP032666919, [retrieved on 20141021], DOI: 10.1109/ ULTSYM.2014.0451**
• **IVAN Z NENADIC ET AL: "Measuring bladder viscoelasticity using ultrasound", ULTRASONICS SYMPOSIUM (IUS), 2012 IEEE INTERNATIONAL, IEEE, 7 October 2012 (2012-10-07), pages 105 - 108, XP032434588, ISSN: 1948-5719, ISBN: 978-1-4673-4561-3, DOI: 10.1109/ULTSYM.2012.0026**
• **IVAN Z NENADIC ET AL: "Paper;Ultrasound bladder vibrometry method for measuring viscoelasticity of the bladder wall;Ultrasound bladder vibrometry method for measuring viscoelasticity of the bladder wall", PHYSICS IN MEDICINE AND BIOLOGY, INSTITUTE OF PHYSICS PUBLISHING, BRISTOL GB, vol. 58, no. 8, 3 April 2013 (2013-04-03), pages 2675 - 2695, XP020239452, ISSN: 0031-9155, DOI: 10.1088/ 0031-9155/58/8/2675**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DAVID MARLEVI ET AL: "Plaque characterization using shear wave elastography-evaluation of differentiability and accuracy using a combinedandsetup", 23 November 2018, PHYSICS IN MEDICINE AND BIOLOGY, INSTITUTE OF PHYSICS PUBLISHING, BRISTOL GB, PAGE(S) 235008, ISSN: 0031-9155, XP020332191

**Description**

CROSS-REFERENCE TO RELATED APPLICATIONS

This application claims the benefit of U.S. Provisional Patent Application Serial No. 63/215,912 filed on June 28, 2021 and entitled "System and Method for Non-Invasive Determination of Pressure in a Biological Compartment".

STATEMENT REGARDING FEDERALLY SPONSORED RESEARCH

N/A

BACKGROUND

Biological compartments that sustain internal fluid pressure are ubiquitous structures in mammalian organisms. Various scaling relationships between anatomical structures of this character in different species can be understood in relation to their mechanical role as pressurized compartments. In relation to human health and medicine, the pressures that such compartments are exposed to are often vital indicators of their functioning. Examples, where pressure measurements hold clinical significance in biological compartments include the diaphragm and lungs, large blood vessels, the heart, the bladder, and body cavities.

Pressure measurements are typically collected by invasive procedures that use catheters instrumented with pressure transducers. Because of the potential for discomfort and morbidity is high in such procedures, there is great interest toward non-invasive assessments.

Due to the sensitivity of shear wave speed to tissue loading [11], wave-based elastographic techniques (i.e. shear wave and Lamb wave elastography) are increasingly being investigated as a noninvasive means of inferring loads sustained by soft tissues. Recently, a variety of investigations have correlated internal fluid pressures with transverse wave speeds at the walls of a variety of biological compartments. However, these investigations are empirical and the underlying mechanics that produce such associations has received limited treatment. An analysis that relates elastic wave speed to pressure in such structures would need to incorporate analyses for acoustoelastic wave propogation in the presence of finite deformation in a nonlinear and hyperelastic solid; wave dispersion associated with the wall thickness of the compartment; and pressure-vessel analysis for highly deformed compartments. None of these criteria have been applied toward estimation of pressure in a biological compartment. Thus there remains a need for a non-invasive method for measuring pressure, such as in a bladder.

SUMMARY OF THE DISCLOSURE

The invention is defined in the appended set of claims. The present disclosure addresses the aforementioned drawbacks by providing systems and methods for a mechanical analysis for determining pressure from ultrasonically measured Lamb wave speed in a pressurized biological compartment. In some configurations, the method includes acoustoelastic analysis of Lamb waves with spherical pressure-vessel analysis. The systems and methods may provide for non-invasive pressure estimation in biological compartments which is relevant to a range of biological structures.

In one configuration, a method is provided for determining a kinetic value of a tissue volume. The method includes, with a transducer, generating Lamb waves in a tissue wall of the tissue volume using radiation force. The tissue volume is formed by a tissue wall that spatially separates a fluid material from a rigid material. The method also includes forming ultrasonic echo data by detecting ultrasonic energy reflected by multiple locations along the tissue volume that is subject to the radiation force. The method also includes determining frequency-resolved wave speed data for the generated Lamb waves from the ultrasonic echo data and determining pressure of the tissue volume based upon the determined frequency-resolved wave speed data.

In one configuration, a system is provided for determining a kinetic value of a tissue volume. The system includes a transducer configured to generate Lamb waves in a tissue wall of the tissue volume using radiation force and detect ultrasonic energy reflected by multiple locations along the tissue volume that is subject to the radiation force to form ultrasonic echo data. The tissue volume is formed by the tissue wall that spatially separates a fluid material from a rigid material. The system also includes a computer system configured to: determine frequency-resolved wave speed data from the generated Lamb waves from the ultrasonic echo data; and determine pressure of the tissue volume based upon the determined frequency-resolved wave speed data. US 2014/296709 A1 teaches systems and methods for analyzing tissue structure and function.

IVAN Z NENADIC ET AL: "Ultrasound bladder vibrometry method for measuring viscoelasticity of the bladder wall", PHYSICS IN MEDICINE AND BIOLOGY, INSTITUTE OF PHYSICS PUBLISHING, BRISTOL GB, vol. 58, no. 8, 3 April 2013 (2013-04-03), pages 2675-2695, ISSN: 0031-9155, DOI: 10.1088/0031-9155/58/8/2675, introduces ultrasound bladder vibrometry (UBV), a method for rapid and noninvasive measurement of bladder wall viscoelasticity.

The foregoing and other aspects and advantages of the present disclosure will appear from the following description. In the description, reference is made to the accompanying drawings that form a part hereof, and in which there is shown by way of illustration a preferred embodiment. This embodiment does not necessarily represent the full scope of the invention, however, and reference is therefore made to the claims and herein for interpreting the scope of the invention. Like reference numerals will be used to refer to like parts from Figure to Figure in the following description.

BRIEF DESCRIPTION OF THE DRAWINGS

FIG. 1 is a block diagram of an ultrasound imaging system used in accordance with the present disclosure.
FIG. 2 is a block diagram of a transmitter that forms part of the ultrasound imaging system of FIG. 1.
FIG. 3 is a block diagram of a receiver that forms part of the ultrasound imaging system of FIG. 1.
FIG. 4A is a flow chart of non-limiting example steps for of a method to determine kinetic values from ultrasonically measured Lamb wave speed in a pressurized biological compartment.
FIG. 4B is a flow chart of non-limiting example steps for of a method to determine pressure from ultrasonically measured Lamb wave speed in a pressurized biological compartment.
FIG. 5A is a diagram of the basic principal of ultrasound bladder vibrometry (UBV) of the present disclosure.
FIG. 5B illustrates *in vivo* human B-mode of the bladder with a sketch of the UBV excitation beam.
FIG. 6A depicts a non-limiting example compartment modeled as a hyperelastic spherical pressure vessel with Lamb waves propagating along the circumference.
FIG. 6B depicts the acoustic pulse producing Lamb waves propagating forward and backward away from the pulse of FIG. 6A.
FIG. 6C depicts a 2D-FFT based method used to estimate frequency dependent phase velocities from the waves of FIG. 6B.
FIG. 6D depicts a non-limiting example curve fitting applied to the median wave speeds from the acquisitions collected at each volume of FIG. 6A.
FIG. 7 shows graphs of non-limiting examples of vessel pressures plotted along with the vessel pressure measured by a pressure gauge as a function of bladder volume.
FIG. 8 is a correlation plot of measurements from a non-limiting example set of bladders.
FIG. 9 is a graph of a non-limiting example signal and noise region of interest (ROI).

DETAILED DESCRIPTION

Systems and methods are provided for a mechanical analysis for determining kinetic values, such as pressure, stress, and the like, from ultrasonically measured Lamb wave speed in a pressurized biological compartment. In some configurations, the method includes acoustoelastic analy5sis of Lamb waves with spherical pressure-vessel analysis. In a non-limiting example, bladder pressure may be determined with varied filling volumes and elasticities. The systems and methods may provide for non-invasive pressure estimation in biological compartments which is relevant to a range of biological structures.

"Kinetic value" is a quantity relating to the state of the forces acting on the material such as pressure, stress and/or force.

A mechanical analysis may be used that directly relates the pressure in a spherical pressure-vessel to measurements of the dispersion curve of a Lamb wave. Pressure may be determined from UBV measurements collected from the biological compartment, such as the bladder.

Conventional methods rely upon simple empirical association or curve fitting for the purpose of pressure estimation in biological compartments. The systems and methods in accordance with the present disclosure may be generalized to compartments of varied characteristics (e.g. elasticity, size, and the like) as long as the characteristics are included within the mechanistic analysis. The systems and methods in accordance with the present disclosure also include greater accuracy than conventional methods. Deviations in individual bladders suggest that additional effects may be affecting the measurement. For a mechanistic analysis, greater accuracy may be attained by examining the simplifying assumptions in the analysis and adapting the analysis and measurements to incorporate additional characteristics. Non-limiting examples of characteristics that may be modified include adjusting a spherical compartment to a non-spherical geometry that may alter the relation between wall-stress and internal pressure.

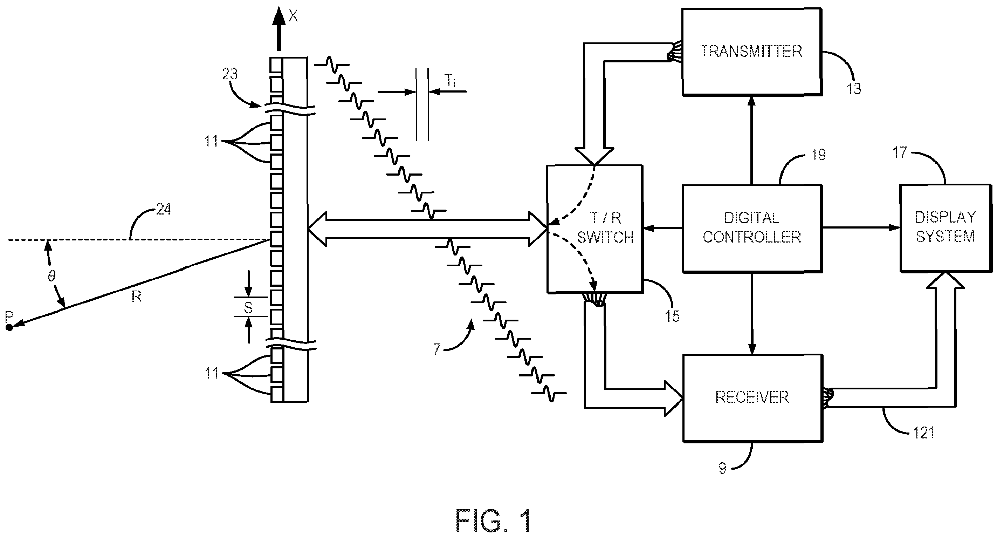

Referring to FIG. 1, a transducer array 23 includes a plurality of separately driven elements 11 that each produce a burst of ultrasonic energy when energized by a pulse produced by a transmitter 13. The ultrasonic energy reflected back to the transducer array 23 from the subject under study is converted to an electrical signal by each transducer element 11 and applied separately to a receiver 9 through a set of switches 15. The transmitter 13, receiver 9 and the switches 15 are operated under the control of a digital controller 19 responsive to the commands input by the human operator. A complete scan is performed by acquiring a series of echoes in which the switches 15 are set to their transmit position, the transmitter 13 is gated on momentarily to energize each transducer element 11, the switches 15 are then set to their receive position, and the subsequent echo signals produced by each transducer element 11 are applied to the receiver 9. The separate echo signals from each transducer element 11 are combined in the receiver 9 to produce a single echo signal that is employed to produce a line in an image on a display system 17.

The transmitter 13 drives the transducer array 23 such that the ultrasonic energy produced is directed, or steered, in a beam or pulse. A B-scan can therefore be performed by moving this beam through a set of angles from point-to-point rather than physically moving the transducer array 23. To accomplish this, the transmitter 13 imparts a time delay (Ti) to the respective pulses 20 that are applied to successive transducer elements 11. If the time delay is zero (Ti = 0), all the transducer elements 11 are energized simultaneously and the resulting ultrasonic beam is directed along an axis 24 normal to the transducer face and originating from the center of the transducer array 23. As the time delay (Ti) is increased, the ultrasonic beam is directed downward from the central axis 24 by an angle $\theta$.

A sector scan is performed by progressively changing the time delays Ti in successive excitations. The angle $\theta$ is thus changed in increments to steer the transmitted beam in a succession of directions. When the direction of the beam is above the central axis 24, the timing of the pulses 7 is reversed.

Referring still to FIG. 1, the echo signals produced by each burst of ultrasonic energy emanate from reflecting objects located at successive positions (R) along the ultrasonic beam. These are sensed separately by each segment 11 of the transducer array 23 and a sample of the magnitude of the echo signal at a particular point in time represents the amount of reflection occurring at a specific range (R). Due to the differences in the propagation paths between a focal point P and each transducer element 11, however, these echo signals will not occur simultaneously and their amplitudes will not be equal. The function of the receiver 9 is to amplify and demodulate these separate echo signals, impart the proper time delay to each and sum them together to provide a single echo signal that accurately indicates the total ultrasonic energy reflected from each focal point P located at range R along the ultrasonic beam oriented at the angle $\theta$.

To simultaneously sum the electrical signals produced by the echoes from each transducer element 11, time delays are introduced into each separate transducer element channel of the receiver 9. In the case of the linear array 23, the delay introduced in each channel may be divided into two components, one component is referred to as a beam steering time delay, and the other component is referred to as a beam focusing time delay. The beam steering and beam focusing time delays for reception are precisely the same delays (Ti) as the transmission delays described above. However, the focusing time delay component introduced into each receiver channel is continuously changing during reception of the echo to provide dynamic focusing of the received beam at the range R from which the echo signal emanates.

Under the direction of the digital controller 19, the receiver 9 provides delays during the scan such that the steering of the receiver 9 tracks with the direction of the beam steered by the transmitter 13 and it samples the echo signals at a succession of ranges and provides the proper delays to dynamically focus at points P along the beam. Thus, each emission of an ultrasonic pulse results in the acquisition of a series of data points that represent the amount of reflected sound from a corresponding series of points P located along the ultrasonic beam.

By selecting proper time delays, echoes from multiple focused locations can be received to measure vibration information from several points of the tissue. The limitation of the lateral resolution of the transducer for two closely located points can be improved by assigning different transmitting codes for different locations.

The display system 17 receives the series of data points produced by the receiver 9 and converts the data to a form producing the desired image. For example, if an A-scan is desired, the magnitude of the series of data points is merely graphed as a function of time. If a B-scan is desired, each data point in the series is used to control the brightness of a pixel in the image, and a scan comprised of a series of measurements at successive steering angles ($\theta$) is performed to provide the data necessary for display of an image.

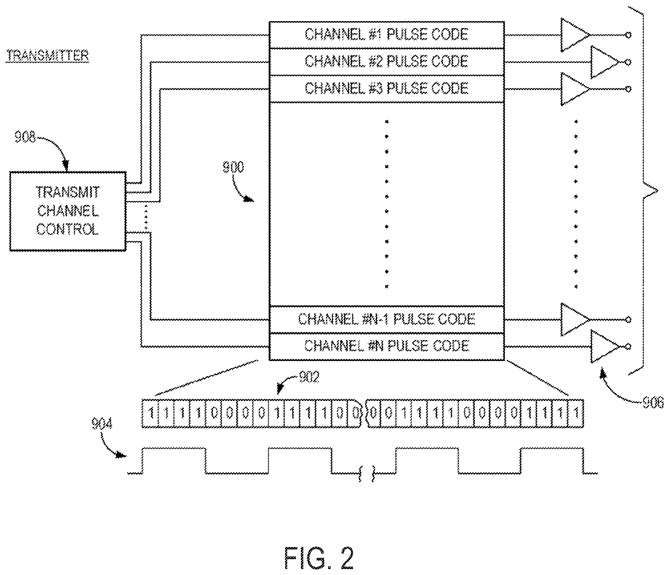

Referring to FIG. 2, the transmitter 13 includes a set of channel pulse code memories that are indicated collectively at 900. Each pulse code memory 900 stores a bit pattern 902 that determines the frequency of the ultrasonic pulse 904 that is to be produced. This bit pattern is read out of each pulse code memory 900 by a master clock and applied to a driver 906 that amplifies the signal to a power level suitable for driving the transducer 23. In the example shown in FIG. 2, the bit pattern is a sequence of four "1" bits alternated with four "0" bits to produce a 5 megahertz ("MHz") ultrasonic pulse 904. The transducer elements 23 to which these ultrasonic pulses 904 are applied respond by producing ultrasonic energy.

As indicated above, to steer the transmitted beam of the ultrasonic energy in the desired manner, the pulses 904 for each of the N channels must be produced and delayed by the proper amount. These delays are provided by a transmit control 908 that receives control signals from the digital controller 19. When the control signal is received, the transmit control 908 gates a clock signal through to the first transmit channel 900. At each successive delay time interval thereafter, the clock signal is gated through to the next channel pulse code memory 900 until all the channels to be energized are producing their ultrasonic pulses 904. Each transmit channel 900 is reset after its entire bit pattern 902 has been transmitted and the transmitter 13 then waits for the next control signal from the digital controller 19.

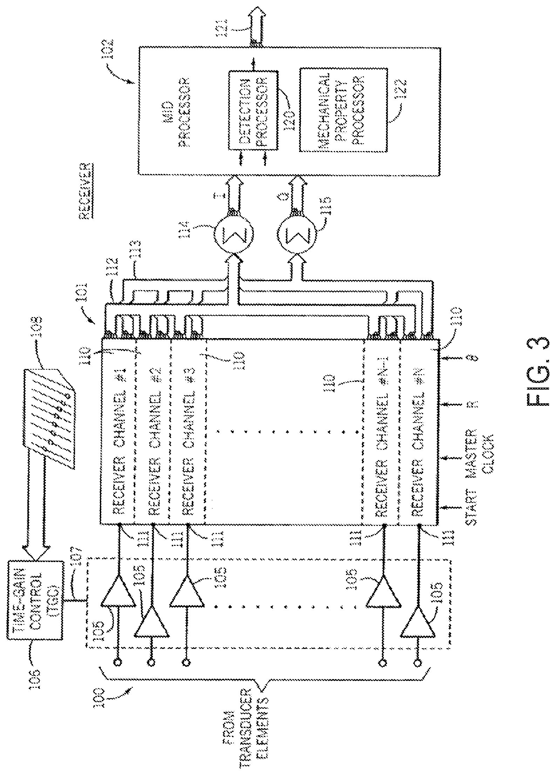

Referring to FIG. 3, the receiver 9 is comprised of three sections: a time-gain control section 100, a beam forming section 101, and a mid processor 102. The time-gain control section 100 includes an amplifier 105 for each of the N=128 receiver channels and a time-gain control circuit 106. The input of each amplifier 105 is connected to a respective one of the transducer elements 11 to receive and amplify the echo signal that it receives. The amount of amplification provided by the amplifiers 105 is controlled through a control line 107 that is driven by the time-gain control circuit 106. As is well known in the art, as the range of the echo signal increases, its amplitude is diminished. As a result, unless the echo signal emanating from more distant reflectors is amplified more than the echo signal from nearby reflectors, the brightness of the image diminishes rapidly as a function of range (R). This amplification is controlled by the operator who manually sets TGC linear potentiometers 108 to values that provide a relatively uniform brightness over the entire range of the sector scan. The time interval over which the echo signal is acquired determines the range from which it emanates, and this time interval is divided into by the TGC control circuit 106. The settings of the potentiometers are employed to set the gain of the amplifiers 105 during each of the respective time intervals so that the echo signal is amplified in ever increasing amounts over the acquisition time interval.

The beam forming section 101 of the receiver 9 includes N=128 separate receiver channels 110. Each receiver channel 110 receives the analog echo signal from one of the TGC amplifiers 105 at an input 111, and it produces a stream of digitized output values on an I bus 112 and a Q bus 113. Each of these I and Q values represents a sample of the echo signal envelope at a specific range (R). These samples have been delayed in the manner described above such that when they are summed at summing points 114 and 115 with the I and Q samples from each of the other receiver channels 110, they indicate the magnitude and phase of the echo signal reflected from a point P located at range R on the steered beam ($\theta$).

Referring still to FIG. 3, the mid processor section 102 receives the beam samples from the summing points 114 and 115. The I and Q values of each beam sample is a 16-bit digital number that represents the in-phase and quadrature components of the magnitude of the reflected sound from a point (R,$\theta$). The mid processor 102 can perform a variety of calculations on these beam samples, where choice is determined by the type of image to be reconstructed.

For example, a conventional ultrasound image may be produced by a detection processor 120 that calculates the magnitude of the echo signal from its I and Q components:

$$M = \sqrt{I^2 + Q^2}$$

The resulting magnitude values output at 121 to the display system 17 result in an image in which the magnitude of the reflected echo at each image pixel is indicated.

The present disclosure may be implemented by a mechanical property processor 122 that forms part of the mid-processor 102. As will be explained in detail below, this processor 102 receives the I and Q beam samples acquired during a sequence of measurements of the subject tissue and calculates a mechanical property (i.e., thickness) of the tissue.

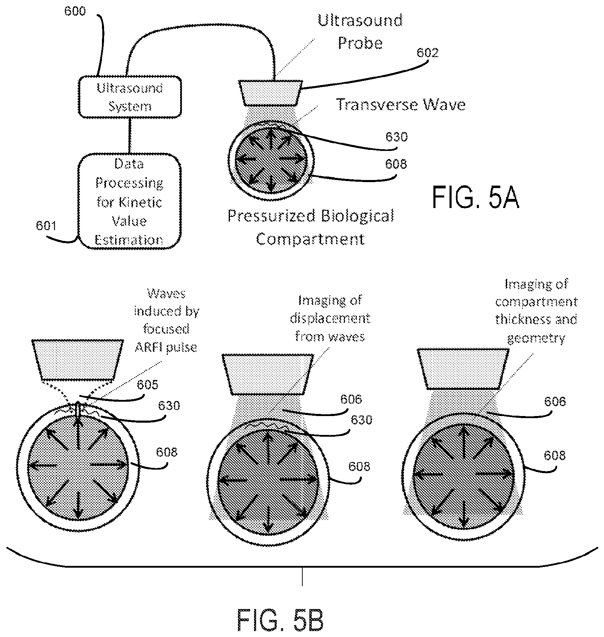

Referring to FIG. 5A and FIG. 5B, a focused ultrasound beam 605 may be used to induce an acoustic radiation force that excites waves 630 in the wall of a compartment 608 and a pulse-echo detection beam 606 may be used to measure the motion at several points along a line of propagation in order to track tissue motion through successive B-modes. The line of propagation being, for example, a bladder wall. Though the following description is made with respect to the bladder wall, other elastic tissue volumes may likewise be considered. The B-mode scanning can be performed by, for example, a linear array transducer 602 with ultrasound system 600 and a data processing module for kinetic value estimation 601, as shown in FIG. 5A. The detection beam 606 can be transmitted with a pulse repetition frequency (PRF). FIG. 5B illustrates a B-mode cross-section of a non-limiting example compartment.

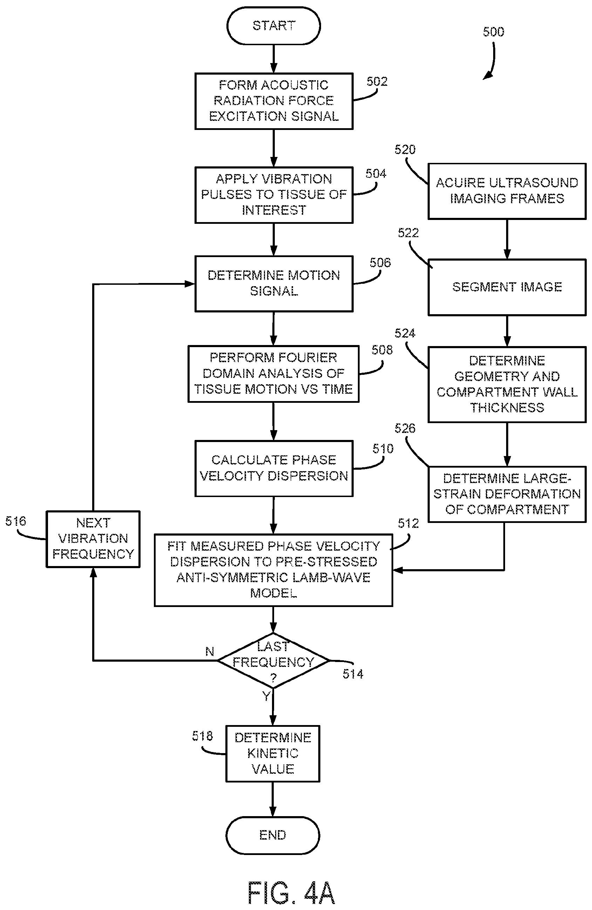

Referring to FIG. 4A, a flow chart is provided setting forth example steps 500 of a method to determine a kinetic value of a biological compartment, such as pressure, tensile stress, and the like. The process begins at process block 502 with the formation of acoustic radiation force excitation signals that are applied, at process block 504, to a subject to, thereby, apply vibration pulses to a tissue wall of a tissue of interest.

At process block 506, a pulse-echo ultrasound detection beam is used to measure the motion at several points along a line of propagation, the line of propagation being, for example, a bladder wall, in order to track tissue motion through successive B-modes. Cross-spectral analysis of the received echoes can then be used to calculate the wall motion as a function of time. Because an impulse contains mostly the frequency components up to the inverse of the time length of the impulse, many phase velocities from a single impulse push can be extracted.

As shown at process block 508, a two-dimensional fast Fourier transform (2D-FFT) of the wall motion may be used to calculate the change of Lamb wave velocity as a function of frequency, or the Lamb wave dispersion. In other words, Fourier-space analysis of the motion may be used as a function of time that yields the k-space whose coordinates are frequency, $f$, and wave number, $k$.

Since the wave velocity $c = k/f$, the phase velocity at each frequency can be calculated at process block 510 by searching for peaks at the given frequency and dividing by the wave number coordinate by the frequency coordinate for the given peak. The dispersion data may be fit to the pre-stressed anti-symmetric Lamb wave model at process block 512, to determine a kinetic value (such as pressure, stress, and the like), as shown at process block 518. As indicated by process block 514, the digital controller determines whether the last frequency has been measured when a harmonic excitation approach is used. If not, at process block 516, another frequency is selected and process blocks 506 through 512 are repeated at each desired prescribed frequency.

Referring still to FIG. 4A, an ultrasound deformation estimation may be performed where ultrasound imaging frames are acquired at process block 520. The resulting image may be segmented at process block 522. The geometry of the compartment and the wall thickness may be determined at process block 524. Large-strain deformation of the compartment may be determined at process block 526, which may be fed into process block 512.

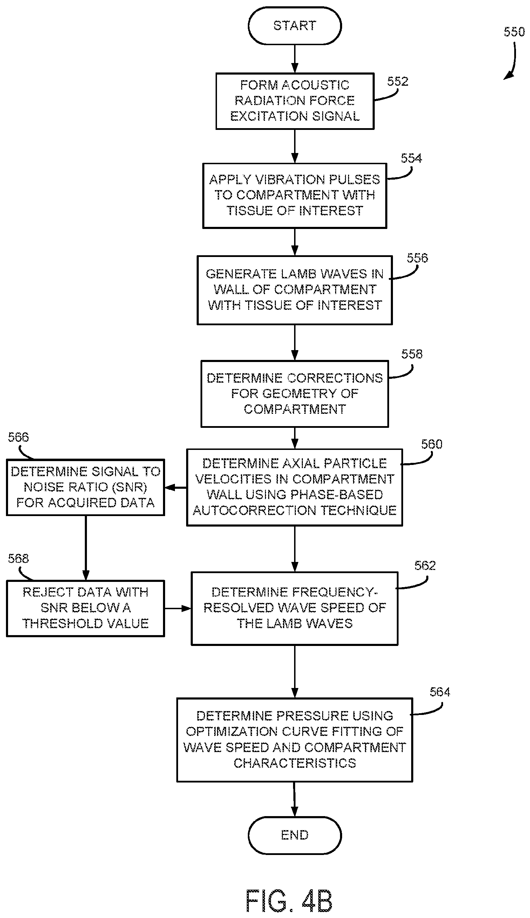

Referring now to FIG. 4B, a flow chart is provided setting forth non-limiting example steps 550 of a method to determine pressure from ultrasonically measured Lamb wave speed in a pressurized biological compartment. An ultrasound system may be used to form an acoustic radiation force excitation signal at step 552. Using the radiation force excitation signal, vibration pulses may be applied to a compartment with tissue of interest of a subject at step 554. Lamb waves may be generated therefrom in the wall of the compartment at step 556. Any necessary corrections for the geometry of the compartment may be determined at step 558, such as adjusting for the curvature of a bladder wall, and the like.

Axial particle velocities in the compartment wall may be determined using phase-based autocorrection at step 560. Frequency-resolved wave speed of the Lamb waves may be determined using a 2D Fourier transformation of the particle velocities at step 562. Pressure of the compartment may be determined using an optimization curve fitting of an expression that includes wave speed and compartment characteristics at step 564. An expression may include a dispersion relation that describes wave speed as a function of pressure. In a non-limiting example, the expression may be of the form of eq. (26) below. Characteristics may include compartment geometry, solid and fluid wave densities, and the like. A signal to noise ratio (SNR) may optionally be determined for the acquired data at step 566 and any data below a threshold SNR value may be excluded from the analysis at step 568.

Expressions of stress and deformation in terms of principle stress and stretches and associated stretch invariants may be used in a mechanical analysis. The spectral decomposition of the deformation tensor F can be expressed as

$$F = RU = VR = \sum_{i=1}^{3} \lambda_i v^{(i)} \otimes u^{(i)} = \sum_{i=1}^{3} \lambda_i Ru^{(i)} \otimes u^{(i)} \qquad (1)$$

Here, R represents the orthogonal tensor associated with rigid body rotation, U is the right stretch tensor and, $\lambda_i$ denotes the ith principle stretch and $u^{(i)}$ and $v^{(i)}$ denotes associated unit vector of the principle stretch in the respective material and spatial (e.g. undeformed and deformed) configurations of the medium. In some configurations, $v^{(i)} = Ru^{(i)}$. $\otimes$ denotes a diadic product of two vectors. To remove the influence of R from the deformation measure, constitutive equations may be formulated in terms of the right and left Cauchy-Green deformation tensors, i.e. $C = F^T F = U^2$ and $B = FF^T = V^2$, the latter of which may include the following spectral decomposition and invariants:

$$B = \sum_{i=1}^{3} \lambda_i^2 v^{(i)} \otimes v^{(i)} \qquad (2)$$

$$I_1 = tr(B) = tr(B) = \lambda_1^2 + \lambda_2^2 + \lambda_3^2 \qquad (3)$$

$$I_2 = 1/2\left(tr(B)^2 - tr(B^2)\right) = \lambda_1^2 \lambda_2^2 + \lambda_2^2 \lambda_3^2 + \lambda_1^2 \lambda_3^2 \qquad (4)$$

$$I_3 = \det(B) = \lambda_1^2 \lambda_2^2 \lambda_3^2 \qquad (5)$$

In the case of incompressibility or isochoric motion, $I_3 = 1$. For an isotropic material, the Cauchy stress ($\sigma$) may be co-axial with B and V. The principle Cauchy stresses can be expressed relative to the principle stretches and principle vectors of V and a strain energy function W. Adding in the additional constraint of incompressibility results in the following expression:

$$\sigma = \sum_{i=1}^{3} \lambda_i \frac{\partial W}{\partial \lambda_i} v^{(i)} \otimes v^{(i)} - pI \qquad (6)$$

where, p is a Lagrangian multiplier that may be added to enforce the incompressiblity constraint.

*Non-Limiting Example Spherical Pressure Vessel Model of the Bladder*

Relating wall stresses to vessel pressure, the biological compartment may be modeled as a spherical, thin-walled pressure vessel. The classic relation for Cauchy stress along the circumference of the sphere (set to the first principle Cauchy stress $\sigma_1$) is given by:

$$\sigma_1 = \frac{\mathrm{Pr}}{2t} = \frac{\mathrm{Pr}}{4h} \qquad (7)$$

where P represents the gauge pressure relative to the exterior vessel wall and r, t and h are the radius, wall thickness and half-wall thickness of the deformed sphere. $\sigma_2$, the principle Cauchy stress in the radial direction, takes the following values at the boundaries of the vessel walls

$$\sigma_2 = \begin{cases} -P \text{ at the interior vessel wall} \\ 0 \text{ at the exterior vessel wall} \end{cases} \qquad (8)$$

If r/4h >>1, which is the case in a thin-walled vessel, the difference in the wall stresses can be approximated about the thickness of the vessel wall by

$$\sigma_1 - \sigma_2 \approx P\frac{r}{4h} \qquad (9)$$

In the case of incompressiblity, the principle stretches can be defined by the vessel radius r relative to its initial value $r_0$. They can then, in turn, be determined from the initial and deformed volumes V and $V_0$ via $r = (3/4V/\pi)^{1/3}$. These relations may be of the forms:

$$\lambda_1 = \frac{r}{r_0} = \left(\frac{V}{V_0}\right)^{1/3} \qquad (10)$$

$$\lambda_2 = \frac{r_0^2}{r^2} = \left(\frac{V_0}{V}\right)^{2/3} \qquad (11)$$

The initial fill volume $V_0$ may be difficult to acquire practically. In some configurations to address this challenge, $V_0$ may be determined from deformed and undeformed tissue wall thickness as: $V_0 = \left(\frac{h}{h_0}\right)^{3/2} V$ , where h is the deformed tissue wall thickness, and $h_0$ is the undeformed tissue wall thickness.

*Lamb Wave Propogation in a Pre-Stressed Plate*

In some configurations, a Lamb wave analysis may be performed. An expression for Lamb waves propagating in a pre-stressed, isotropic, incompressible and hyperelastic plate surrounded by fluid is provided below in eq. (12), which is a dispersion equation for axisymmetric transverse waves propogating in such a condition

$$\gamma s_1 \left(1 + s_2^2\right)^2 \tanh\left(s_1 kh\right) - \gamma s_2 \left(1 + s_1^2\right)^2 \tanh\left(s_2 kh\right) + \frac{\rho^F c^2}{\xi}\left(s_1^2 - s_2^2\right) = 0$$

$$(12)$$

where h is half the thickness of the deformed hyperelastic plate (i.e. $h = \lambda_2 h_0$), c is the wave speed, $\rho^F$ is the mass density of the fluid (assumed to be 1 g/ml). $\xi$ is a parameter defined by $\xi = \sqrt{1 - \dfrac{c^2}{c_p^2}}$ where c is the speed of the transverse wave

and $c_p$ is the speed of sound in the fluid (assumed to be 1480 m/s in this current analysis). k is the wave number and it defines the frequency dependence of the wave speed through the relation $k = 2\pi f/c$. $s_1^2$ and $s_2^2$ are roots of the quadratic equation defined by

$$\gamma s^4 - \left(2\beta - \rho c^2\right)s^2 + \alpha - \rho c^2 = 0 \qquad (13)$$

Here, $\rho$ represents the mass density of the solid wall, which in some configurations may be assumed to be 1 g/ml. $\gamma$, $\alpha$ and $\beta$ are the acoustoelastic parameters for a pre-stressed hyperelastic solid. They may be defined by the components of the incremental elasticity tensor $A_{0piqj} = F_{pr}F_{qs}(\partial^2 W/\partial F_{ir}\partial F_{js})$. Using relationships derived between $A_{0piqj}$, W and $\lambda_i$, the following expressions may be generated

$$\alpha = A_{01212} = \frac{\lambda_1^2 \left(\lambda_1 \dfrac{\partial W}{\partial \lambda_1} - \lambda_2 \dfrac{\partial W}{\partial \lambda_2}\right)}{\lambda_1^2 - \lambda_2^2} \qquad (14)$$

$$\gamma = A_{02121} = \frac{\lambda_2^2 \left(\lambda_1 \dfrac{\partial W}{\partial \lambda_1} - \lambda_2 \dfrac{\partial W}{\partial \lambda_2}\right)}{\lambda_1^2 - \lambda_2^2} \qquad (15)$$

$$2\beta = A_{01111} + A_{02222} - 2A_{01122} - 2A_{01221} = \lambda_1^2 \frac{\partial^2 W}{\partial \lambda_1^2} + \lambda_2^2 \frac{\partial^2 W}{\partial \lambda_2^2} - 2\lambda_1\lambda_2 \frac{\partial^2 W}{\partial \lambda_2 \lambda_1} - 2\frac{\lambda_1\lambda_2\left(\lambda_2 \dfrac{\partial W}{\partial \lambda_1} - \lambda_2 \dfrac{\partial W}{\partial \lambda_2}\right)}{\lambda_1 - \lambda_2}$$

$$(15)$$

Here, $\lambda_1$ may be aligned with the circumferential direction of the vessel wall while $\lambda_2$ is in the radial direction. These expressions are a consequence of the same co-axility between stress and strain measures and so material isotropy may be assumed in the expressions. A substitution of the components of the principle Cauchy stresses (e.g. $\sigma_i = \lambda_i \partial W/\partial \lambda_i$) from eq. (6) into eq. (14) and eq. (15) produces

$$\alpha = \frac{\lambda_1^2}{\lambda_1^2 - \lambda_2^2}\left(\sigma_1 - \sigma_2\right) \qquad (16)$$

$$\gamma = \frac{\lambda_2^2}{\lambda_1^2 - \lambda_2^2}\left(\sigma_1 - \sigma_2\right) \qquad (17)$$

Eq. (17) and eq. (18) are model-independent, but substitutions may not be made for β without producing derivatives of the components of σ. In some configurations, a constructive model may be used to determine the wall stress. In a non-limiting example, the Demiray-Fung constitutive model may be used to produce an expression for β of a similar form as that of eq. (17) and eq. (18). A strain energy function can be expressed in terms of the first invariant of B (e.g. eq. (3)) as follows

$$W = \frac{\mu_0}{2b}\left(e^{b(I_1-3)} - 1\right) \tag{18}$$

Where $\mu_0$ represents the initial shear modulus of the medium and b is a unitless parameter that determines the rate of strain stiffening within the medium. Substituting eq. (19) into eq. (16) produces the following expression for β for the non-limiting example model:

$$2\beta = 2\left(\lambda_1^4 + \lambda_2^4 - 2\lambda_1^2\lambda_2^2\right)\mu_0 b\left(e^{b(I_1-3)}\right) + 2\left(\lambda_1^2 + \lambda_2^2\right)\mu_0\left(e^{b(I_1-3)}\right) \tag{19}$$

An expression for stresses can be produced by substituting eq. (19) into eq. (6) and subtracting the components to eliminate p, which produces:

$$\sigma_1 - \sigma_2 = \lambda_1\frac{\partial W}{\partial\lambda_1} - \lambda_2\frac{\partial W}{\partial\lambda_2} = \left(\lambda_1^2 - \lambda_2^2\right)\frac{\partial W}{\partial I_1} = \left(\lambda_1^2 - \lambda_2^2\right)\mu_0 e^{b(I_1-3)} \tag{20}$$

An expression for β in terms of stress can be produced by substituting eq. (21) into eq. (20), which results in:

$$2\beta = \frac{\left(\lambda_1^4 + \lambda_2^4 - 2\lambda_1^2\lambda_2^2\right)b + \lambda_1^2 + \lambda_2^2}{\lambda_1^2 - \lambda_2^2}\left(\sigma_1 - \sigma_2\right) \tag{21}$$

The only material parameter remaining is the nonlinearity parameter, b, which may be assumed or estimated. Having γ, α, and β in terms of stress and deformation now permits eq. (12) to be defined in terms of pressure and geometric measures. The principle stretch terms may be encompassed under coefficients, A and B, as follows:

$$A = \frac{\lambda_1^2}{\lambda_1^2 - \lambda_2^2} = \frac{\left(\frac{V}{V_0}\right)^{2/3}}{\left(\frac{V}{V_0}\right)^{2/3} - \left(\frac{V_0}{V}\right)^{4/3}} \tag{22}$$

$$B = \frac{\left(\lambda_1^4 - \lambda_2^4 - 2\lambda_1^2\lambda_2^2\right)b + \lambda_1^2 + \lambda_2^2}{\lambda_1^2 - \lambda_2^2} = \frac{\left(\left(\frac{V}{V_0}\right)^{8/3} - \left(\frac{V_0}{V}\right)^{16/3} - \left(\frac{V}{V_0}\right)^{2/3}\left(\frac{V_0}{V}\right)^{4/3}\right)b}{\left(\frac{V}{V_0}\right)^{2/3} - \left(\frac{V_0}{V}\right)^{4/3}} + \frac{\left(\frac{V}{V_0}\right)^{2/3} + \left(\frac{V_0}{V}\right)^{4/3}}{\left(\frac{V}{V_0}\right)^{2/3} - \left(\frac{V_0}{V}\right)^{4/3}}$$

$$(23)$$

Propagating all substitutions from eq. (17), eq. (18) and eq. (22) into eq. (12) and substituting for $\sigma_1 - \sigma_2$ with eq. (9) produces the non-limiting dispersion equation shown in eq. (25).

$$\frac{V_0^2}{V^2}A\left(P\frac{r}{4h}\right)\sqrt{\frac{\dfrac{\rho c^2}{P\frac{r}{4h}}-2B}{2\frac{V_0^2}{V^2}}+\sqrt{\frac{\left(2B\left(P\frac{r}{4h}\right)-\rho c^2\right)-4\left(\frac{V_0^2}{V^2}A^2\left(P\frac{r}{4h}\right)^2-\frac{V_0^2}{V^2}A\left(P\frac{r}{4h}\right)\rho c^2\right)}{2\frac{V_0^2}{V^2}A\left(P\frac{r}{4h}\right)}}}$$

$$(24)$$

$$\times\left(1+\frac{\dfrac{\rho c^2}{P\dfrac{r}{4h}}-2B}{2\dfrac{V_0^2}{V^2}A}-\frac{\sqrt{\left(2B\left(P\dfrac{r}{4h}\right)-\rho c^2\right)^2-4\left(\dfrac{V_0^2}{V^2}A^2\left(P\dfrac{r}{4h}\right)^2-\dfrac{V_0^2}{V^2}A\left(P\dfrac{r}{4h}\right)\rho c^2\right)}}{2\dfrac{V_0^2}{V^2}A\left(P\dfrac{r}{4h}\right)}\right)^2$$

$$\times\tanh\left(\frac{\dfrac{\rho c^2}{P\dfrac{r}{4h}}-2B}{2\dfrac{V_0^2}{V^2}A}+\frac{\sqrt{\left(2B\left(P\dfrac{r}{4h}\right)-\rho c^2\right)^2-4\left(\dfrac{V_0^2}{V^2}A^2\left(P\dfrac{r}{4h}\right)^2-\dfrac{V_0^2}{V^2}A\left(P\dfrac{r}{4h}\right)\rho c^2\right)}}{2\dfrac{V_0^2}{V^2}A\left(P\dfrac{r}{4h}\right)}kh\right)$$

$$-\frac{V_0^2}{V^2}A\left(P\dfrac{r}{4h}\right)\left(\frac{\dfrac{\rho c^2}{P\dfrac{r}{4h}}-2B}{2\dfrac{V_0^2}{V^2}A}-\frac{\sqrt{\left(2B\left(P\dfrac{r}{4h}\right)-\rho c^2\right)^2-4\left(\dfrac{V_0^2}{V^2}A^2\left(P\dfrac{r}{4h}\right)^2-\dfrac{V_0^2}{V^2}A\left(P\dfrac{r}{4h}\right)\rho c^2\right)}}{2\dfrac{V_0^2}{V^2}A\left(P\dfrac{r}{4h}\right)}\right)$$

$$\times\left(1+\frac{\dfrac{\rho c^2}{P\dfrac{r}{4h}}-2B}{2\dfrac{V_0^2}{V^2}A}+\frac{\sqrt{\left(2B\left(P\dfrac{r}{4h}\right)-\rho c^2\right)^2-4\left(\dfrac{V_0^2}{V^2}A^2\left(P\dfrac{r}{4h}\right)^2-\dfrac{V_0^2}{V^2}A\left(P\dfrac{r}{4h}\right)\rho c^2\right)}}{2\dfrac{V_0^2}{V^2}A\left(P\dfrac{r}{4h}\right)}\right)^2$$

$$\times\tanh\left(\frac{\dfrac{\rho c^2}{P\dfrac{r}{4h}}-2B}{2\dfrac{V_0^2}{V^2}A}-\frac{\sqrt{\left(2B\left(P\dfrac{r}{4h}\right)-\rho c^2\right)^2-4\left(\dfrac{V_0^2}{V^2}A^2\left(P\dfrac{r}{4h}\right)^2-\dfrac{V_0^2}{V^2}A\left(P\dfrac{r}{4h}\right)\rho c^2\right)}}{2\dfrac{V_0^2}{V^2}A\left(P\dfrac{r}{4h}\right)}kh\right)$$

$$+\frac{\rho^F c^2}{\xi}\left(2\frac{\sqrt{\left(2B\left(P\frac{r}{4h}\right)-\rho c^2\right)^2-4\left(\frac{V_0^2}{V^2}A^2\left(P\frac{r}{4h}\right)^2-\frac{V_0^2}{V^2}A\left(P\frac{r}{4h}\right)\rho c^2\right)}}{2\frac{V_0^2}{V^2}A\left(P\frac{r}{4h}\right)}\right)=0$$

Where $h = \lambda_2 h_0 = h_0(V_0/V)^{2/3}$ and $r = (3/4V/\pi)^{1/3}$. Eq. (26) is fully defined in terms of pressure (P), solid and fluid wave densities and wave speeds ($\rho$, $\rho^F$, $c_p$ and c), and compartment geometric measurements ($V_0$, V and $h_0$).

*Non-Limiting Example Ex Vivo Bladder Measurements*

In a non-limiting example, UBV measurements collected from ex vivo pig bladders were used for validation. Nine ex vivo bladders were measured before and after a crosslinking treatment with formalin. One bladder was excluded from the analysis due to a low initial volume (110 ml) that precluded it from inclusion. The remaining bladders had initial filling volumes ranging from 190 to 330 ml. During measurements, a syringe was used to add water to the bladder at 10 ml increments up to a total of 280 ml of added fluid and a pressure gauge was used to measure the vessel pressure of the bladder. A programmable ultrasound system equipped with a linear array ultrasound transducer was used to collect UBV measurements. Lamb waves were excited at the wall of the bladder through acoustic radiation force by a focused ultrasound beam with 400 $\mu$s tone burst. Ultra-fast ultrasound imaging at 4000 frames per second captured the resulting wave propagation. Five acquisitions were collected at each volume.

After adjusting for the curvature of the bladder wall, the axial particle velocities were averaged along the bladder wall and particle velocities were estimated using a phase-based autocorrelation technique. In order to exclude acquisitions with poor signal quality, a wave signal-to-noise calculation was adopted. Each acquisition with a SNR lower than 2 was excluded from analysis. A 2D Fourier transform-based technique was used to estimate the frequency-resolved wave speed of the propagating Lamb wave. To avoid the influence of outlier wave speeds, the median wave speed at each filling volume and frequency was used for curve fitting.

In some configurations, signal filters, such as a median filter and the like, may be applied to the particle velocity data prior to averaging. In some configurations, alternative statistical measures of central tendency, such as medial particle velocity, may be used for averaging.

Estimation of P was accomplished by optimization-based curve fitting applied to eq. (26). Fitting was applied using a simplex search method to find a minimum. If $\Theta_i(P)$ is the value of the left side of eq. (26) for the ith frequency, then P may be estimated by:

$$P^{est} = \arg\min\left\{\sum_{i=1}^{N}\Theta_i P * \overline{\Theta_i(P)}\right\} \qquad (25)$$

The bar above $\Theta_i(P)$ denotes its complex conjugate, which may be used to ensure a real-valued quantity. The initial guess for minimization for P may be any appropriate value, in a the non-limiting example the initial guess was set to 100 mmHg. The frequency range used for fitting was 150-500 Hz. This range was selected in order to match the range used in in-vivo UBV measurements.

The nonlinearity parameter b was set to an assumed value of 5. This is the same value used for modeling vascular tissue and is comparable to the measurement of fibroglandular tissue and ex vivo liver tissue. It is a reasonable assumed value for soft tissue, even if not specialized to bladder tissue in particular. The reference volume (i.e. $V_0$ in eq. (10) and eq. (11)) was set to 150 ml, based on the minimum physiological volumes reported for porcine bladders upon excision.

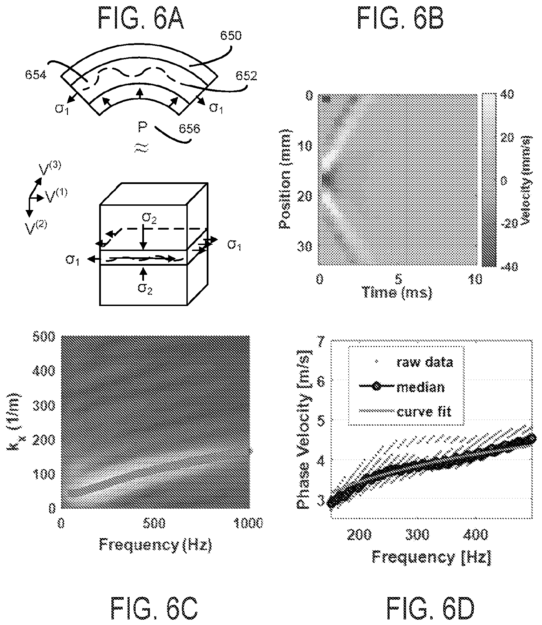

Referring to FIGS. 6A-D, a non-limiting example application to a pressurized biological compartment is shown. FIG. 6A shows the compartment modeled as a hyperelastic spherical pressure vessel 650 with Lamb waves 652 propagating along the circumference 654. Internal pressure P 656 is shown as applying pressure pushing out on hyperelastic spherical pressure vessel 650.

FIG. 6B shows the acoustic pulse producing Lamb waves propagating forward and backward away from the pulse.

FIG. 6C depicts a 2D-FFT based method used to estimate frequency dependent phase velocities from both waves (only a single wave shown).

FIG. 6D shows curve fitting applied to the median wave speeds from the 5 acquisitions collected at each volume.

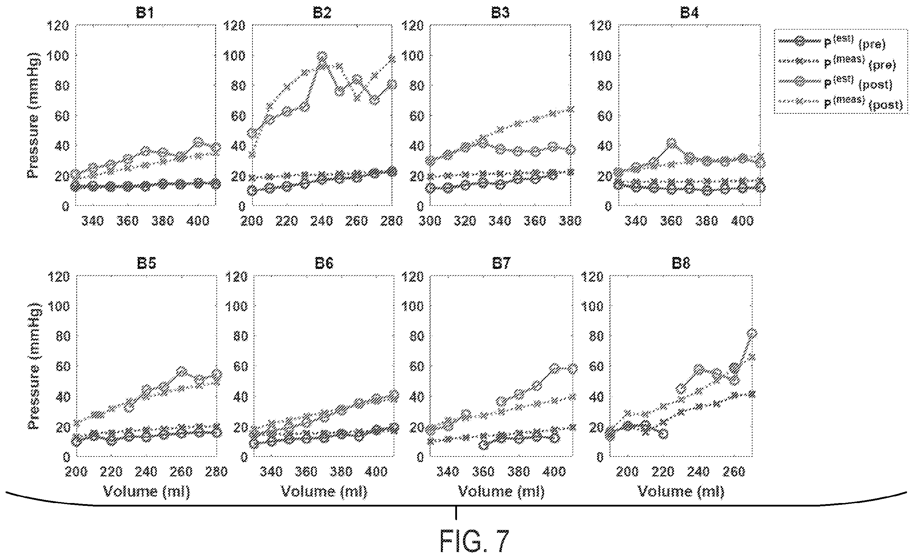

Referring to FIG. 7, non-limiting examples of vessel pressures plotted along with the vessel pressure measured by the pressure gauge as a function of bladder volume are shown. These plots illustrate how the analysis performs at recreating the underlying pressure from wave propagation and deformation (i.e. volume) measurements. Table I reports the corresponding root mean squared deviation (RMSD), mean error (ME) and relative mean error (RME) of estimated pressures relative to measured pressures for the experiments shown in FIG. 7. These quantities are defined respectively as

$$RSMD = \sqrt{1/N \sum_{i=1}^{N} \left( P_i^{(est)} - P_i^{(meas)} \right)^2} \qquad (26)$$

$$ME = 1/N \sum_{i=1}^{N} \left( P_i^{(est)} - P_i^{(meas)} \right) \qquad (27)$$

$$RME = 100 \times \left( 1/N \sum_{i=1}^{N} \left( \frac{\left( P_i^{(est)} - P_i^{(meas)} \right)}{P_i^{(meas)}} \right) \right) \qquad (28)$$

Where the added superscripts denote estimated (est) versus measured (meas) pressures for the ith volume and N is the number of estimates. The estimated pressures were broadly in agreement with the measured pressure (RMSD: 7.90 (mmHg); ME:-1.04 (mmHg); RME: -5.13 (%), though there is considerable variation between bladders (RMSD: 0.58-15.21 (mmHg); ME: -11.77-7.99 (mmHg); RME: - 27.47-21.56 (%))

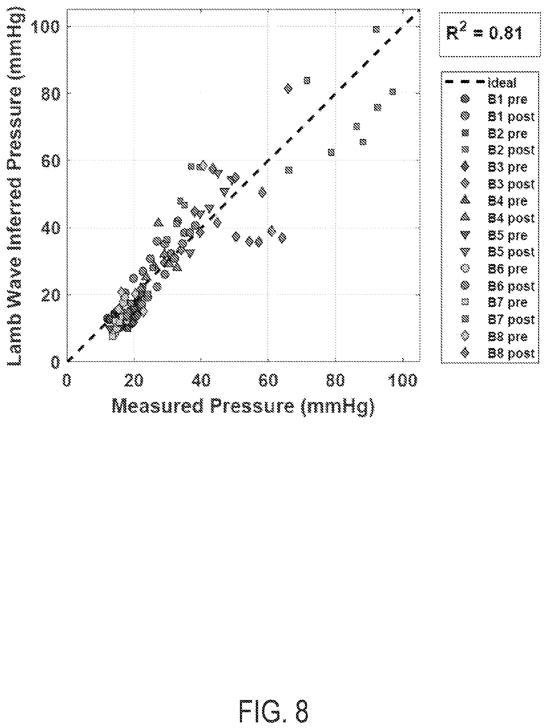

Referring to FIG. 8, a correlation plot of all measurements from the non-limiting example 8 bladders are shown. R2 is calculated as:

$$R^2 = 1 - \frac{\sum_{i=1}^{N} \left( P_i^{(est)} - P_i^{(meas)} \right)^2}{\sum_{i=1}^{N} \left( \left\langle P_i^{(est)} \right\rangle - P_i^{(est)} \right)^2} \qquad (29)$$

Where <> denotes the mean of the quantity. The combined data align with an ideal one-to-one match between measured and inferred vessel pressure (R2 = 0:81).

TABLE I - Descriptive Statistics of the errors in estimated pressures calculated for individual bladders and for all bladders combined. RSMD= root mean squared deviation; ME=mean error; RME=relative mean errors; AND B#= bladder (#).

|  | RSMD (mmHg) | ME (mmHg) | RME[%] |
|---|---|---|---|
| B1 (pre) | 0.579 | 0.01 | 01 0.32 |
| B1 (post) | 5.86 | 5.31 | 20.53 |
| B2 (pre) | 5.13 | -4.35 | -22.09 |
| B2 (post) | 15.21 | -7.1 | -5.22 |
| B3 (pre) | 6.00 | -5.63 | -27.47 |
| B3 (post) | 15.68 | -11.77 | -20.48 |
| B4 (pre) | 4.36 | -4.22 | -26.44 |
| B4 (post) | 5.22 | 1.59 | 6.35 |
| B5 (pre) | 3.91 | -3.78 | -21.87 |
| B5 (post) | 6.03 | 4.07 | 8.78 |

(continued)

| | RSMD (mmHg) | ME (mmHg) | RME[%] |
|---|---|---|---|
| B6 (pre) | 3.42 | -2.43 | -16.33 |
| B6 (post) | 3.35 | -1.84 | -8.64 |
| B7 (pre) | 4.41 | -4.15 | -26.9 |
| B7 (post) | 11.56 | 7.99 | 21.56 |
| B8 (pre) | 8.95 | 2.96 | 7.83 |
| B8 (post) | 9.92 | 4.89 | 7.78 |
| Combined (all) | 7.90 | -1.04 | -5.13 |

Material isotropy and a static fluid may also be assumed in some configurations, such as bladder applications. Other biological compartments, such as large blood vessels and the like, may include other assumptions. Cylindrical geometries may also be used with appropriate modifications to the non-limiting example expressions.

Effects of dynamic loading may also be included, such as by adding additional viscous dispersion and attenuation to the wave propagation. Incorporating the effects of dynamic loading may also produce large strain, viscoelastic stress relaxation.

*Non-limiting Example SNR Calculation*

The signal-to-noise ratio of the propagating Lamb wave may be determined in some configurations, where signal and noise regions of-interests (ROIs) may be collected from the spatiotemporal particle velocity maps. The signal ROI may be collected by fitting a line along the peaks of the propagating waves and collecting an ROI as a window around the linear regression.

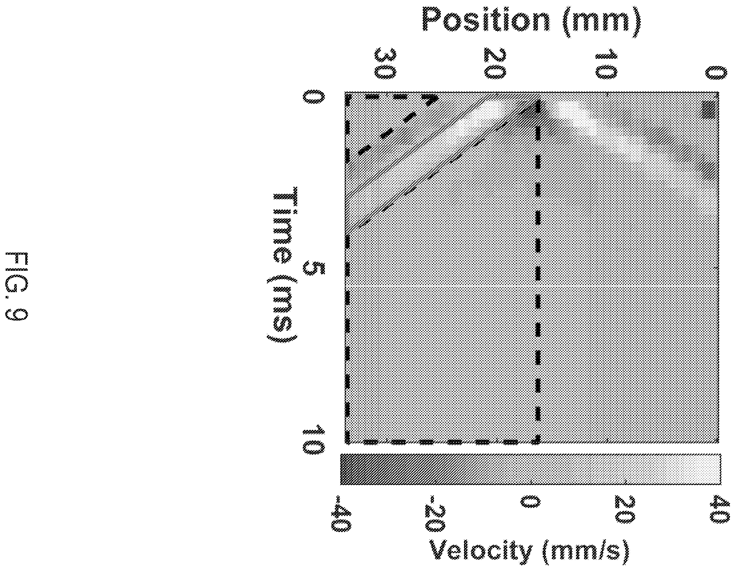

Referring to FIG. 9, non-limiting example signal and noise ROIs are shown. A robust linear fitting may be applied to the time-to-peak arrival times as a function of the lateral location. A noise region-of-interest (ROI) may contain the entire background except for the trough of the propagating wave, such that large peaks or troughs occurring anywhere in the spatiotemporal propagation domain outside of the signal ROI may be included in the noise ROI. Such large peaks act approximately as impulse functions that produce large ripples in the k-space representation of the wave. These ripples can substantially alter the wave speed estimation even when most of the wave propagation has a strong signal.

A time window may also be determined in some configurations. As a non-limiting example, a time window of 1.2 ms may be used to define the signal ROI instead of a spatial window, such as to limit the influence of wavelength variation on the SNR calculation, as wavelength will vary with wave speed at a given frequency. The SNR may then be calculated as:

$$SNR = \frac{\langle S_i \rangle}{std(N_i)} \qquad (30)$$

Where Si and Ni are the ith particle velocity values in the signal and noise ROIs respectively and <> and std() denote the calculated mean and standard deviation

The present disclosure has described one or more preferred embodiments, and it should be appreciated that many alternatives, variations, and modifications, aside from those expressly stated, are possible and within the scope of the invention.

**Claims**

1. A method for determining a kinetic value of a tissue volume, the method comprising:

with a transducer, generating Lamb waves (556) in a tissue wall of the tissue volume using radiation force, wherein the tissue volume is formed by the tissue wall that spatially separates a fluid material from a rigid material;
forming ultrasonic echo data by detecting ultrasonic energy reflected by multiple locations along the tissue volume that is subject to the radiation force;
determining frequency-resolved wave speed data (562) for the generated Lamb waves from the ultrasonic echo data; and

characterized in that the method further comprises determining pressure of the tissue volume directly from the determined frequency-resolved wave speed data. (564)

2. The method of claim 1, wherein determining the kinetic value includes determining pressure and includes optimization-based curve fitting a dispersion relation that includes the frequency-resolved wave speed data.

3. The method of claim 2, wherein the dispersion relation includes a geometry of the tissue volume.

4. The method of claim 3, wherein the geometry is at least one of a sphere or cylinder.

5. The method of claim 4, wherein an undeformed inner volume of the at least one sphere or cylinder is determined from a wall thickness of the tissue wall of the tissue volume.

6. The method of claim 1, further comprising determining axial particle velocities in the tissue wall using a phase-based autocorrelation technique.

7. The method of claim 6, wherein determining frequency-resolved wave speed data includes performing a 2D Fourier transformation of the determined axial particle velocities.

8. The method of claim 6, further comprising determining signal to noise ratios (SNRs) for axial particle velocity data and rejecting axial particle velocity data with SNRs below a determined threshold signal to noise ratio (SNR).

9. The method of claim 1, wherein the tissue volume is a bladder, and the tissue wall is a wall of the bladder.

10. The method of claim 9, further comprising adjusting a dispersion relation for a curvature of the bladder wall.

11. A system for determining a kinetic value of a tissue volume, the system comprising:

a transducer configured to generate Lamb waves (602) in a tissue wall of the tissue volume using radiation force and detect ultrasonic energy reflected by multiple locations along the tissue volume that is subject to the radiation force to form ultrasonic echo data, wherein the tissue volume is formed by the tissue wall that spatially separates a fluid material from a rigid material;
a computer system configured to:

i) determine frequency-resolved wave speed data from the generated Lamb waves from the ultrasonic echo data; (562) and
ii) characterized in that the computer system is further configured to determine a kinetic value of the tissue volume directly from the determined frequency-resolved wave speed data. (518)

12. The system of claim 11, wherein the kinetic value is a pressure and the computer system is further configured to determine the pressure using an optimization-based curve fitting of a dispersion relation that includes the frequency-resolved wave speed data.

13. The system of claim 12, wherein the dispersion relation includes a geometry of the tissue volume.

14. The system of claim 13, wherein the geometry is at least one of a sphere or cylinder.

15. The system of claim 14, wherein the computer system is further configured to determine an undeformed inner volume of the at least one sphere or cylinder from a wall thickness of the tissue wall of the tissue volume.

## Patentansprüche

1. Verfahren zur Bestimmung eines kinetischen Wertes eines Gewebevolumens, wobei das Verfahren umfasst:

Erzeugen, mit einem Wandler, von Lamb-Wellen (556) in einer Gewebewand des Gewebevolumens unter Verwendung von Strahlungskraft, wobei das Gewebevolumen durch die Gewebewand gebildet wird, die ein flüssiges Material räumlich von einem starren Material trennt;

Bilden von Ultraschallechodaten durch Erfassen von Ultraschallenergie, die von mehreren Stellen entlang des Gewebevolumens reflektiert wird, das der Strahlungskraft ausgesetzt ist;
Bestimmen frequenzaufgelöster Wellengeschwindigkeitsdaten (562) für die erzeugten Lamb-Wellen aus den Ultraschallechodaten; und
**dadurch gekennzeichnet, dass** das Verfahren weiterhin umfasst:
Bestimmen des Drucks des Gewebevolumens direkt aus den bestimmten frequenzaufgelösten Wellengeschwindigkeitsdaten (564).

2. Verfahren nach Anspruch 1, wobei das Bestimmen des kinetischen Werts das Bestimmen des Drucks und das optimierungsbasierte Kurvenanpassen einer Dispersionsbeziehung umfasst, die die frequenzaufgelösten Wellengeschwindigkeitsdaten umfasst.

3. Verfahren nach Anspruch 2, wobei die Dispersionsbeziehung eine Geometrie des Gewebevolumens beinhaltet.

4. Verfahren nach Anspruch 3, wobei die Geometrie mindestens eine Kugel oder einen Zylinder umfasst.

5. Verfahren nach Anspruch 4, wobei ein unverformtes Innenvolumen der mindestens einen Kugel oder des mindestens einen Zylinders aus einer Wandstärke der Gewebewand des Gewebevolumens bestimmt wird.

6. Verfahren nach Anspruch 1, das ferner das Bestimmen axialer Partikelgeschwindigkeiten in der Gewebewand unter Verwendung einer phasenbasierten Autokorrelationstechnik umfasst.

7. Verfahren nach Anspruch 6, wobei das Bestimmen frequenzaufgelöster Wellengeschwindigkeitsdaten das Durchführen einer 2D-Fourier-Transformation der bestimmten axialen Teilchengeschwindigkeiten umfasst.

8. Verfahren nach Anspruch 6, das ferner das Bestimmen von Signal-Rausch-Verhältnissen (SNRs) für axiale Partikelgeschwindigkeitsdaten und das Verwerfen von axialen Partikelgeschwindigkeitsdaten mit SNRs unterhalb eines bestimmten Schwellenwert-Signal-Rausch-Verhältnisses (SNR) umfasst.

9. Verfahren nach Anspruch 1, wobei das Gewebevolumen eine Blase ist und die Gewebewand eine Wand der Blase ist.

10. Verfahren nach Anspruch 9, das ferner das Anpassen einer Dispersionsbeziehung für eine Krümmung der Blasenwand umfasst.

11. System zum Bestimmen eines kinetischen Werts eines Gewebevolumens, wobei das System umfasst:

einen Wandler, der so konfiguriert ist, dass er Lamb-Wellen (602) in einer Gewebewand des Gewebevolumens unter Verwendung von Strahlungskraft erzeugt und Ultraschallenergie erfasst, die von mehreren Stellen entlang des Gewebevolumens reflektiert wird, das der Strahlungskraft ausgesetzt ist, um Ultraschallechodaten zu bilden, wobei das Gewebevolumen durch die Gewebewand gebildet wird, die ein flüssiges Material räumlich von einem starren Material trennt;
ein Computersystem, das konfiguriert ist, um:

i) frequenzaufgelöste Wellengeschwindigkeitsdaten aus den erzeugten Lamb-Wellen aus den Ultraschallechodaten zu bestimmen (562); und
**dadurch gekennzeichnet, dass** das Computersystem weiterhin dazu eingerichtet ist,
ii) einen kinetischen Wert des Gewebevolumens direkt aus den ermittelten frequenzaufgelösten Wellengeschwindigkeitsdaten zu bestimmen (518).

12. System nach Anspruch 11, wobei der kinetische Wert ein Druck ist und das Computersystem ferner so konfiguriert ist, dass es den Druck unter Verwendung einer optimierungsbasierten Kurvenanpassung einer Dispersionsbeziehung bestimmt, die die frequenzaufgelösten Wellengeschwindigkeitsdaten umfasst.

13. System nach Anspruch 12, wobei die Dispersionsbeziehung eine Geometrie des Gewebevolumens umfasst.

14. Das System nach Anspruch 13, wobei die Geometrie mindestens eine Kugel oder einen Zylinder umfasst.

15. Das System nach Anspruch 14, wobei das Computersystem ferner so konfiguriert ist, dass es ein unverformtes Innenvolumen der mindestens einen Kugel oder des mindestens einen Zylinders aus einer Wandstärke der Gewebewand des Gewebevolumens bestimmt.

**Revendications**

1. Méthode de détermination d'une valeur cinétique d'un volume tissulaire, la méthode comprenant :

   avec un transducteur, la génération d'ondes de Lamb (556) dans une paroi tissulaire du volume tissulaire à l'aide d'une force de rayonnement, dans laquelle le volume tissulaire est formé par la paroi tissulaire qui sépare spatialement un matériau fluide d'un matériau rigide ;
   la formation de données d'écho ultrasonique en détectant une énergie ultrasonique réfléchie par de multiples emplacements le long du volume tissulaire qui est soumis à la force de rayonnement ;
   la détermination de données de vitesse d'ondes (562) résolues en fréquence pour les ondes de Lamb générées à partir des données d'écho ultrasonique ; et
   **caractérisée en ce que** la méthode comprend en outre la détermination d'une pression du volume tissulaire directement à partir des données de vitesse d'ondes résolues en fréquence déterminées. (564)

2. Méthode selon la revendication 1, dans laquelle la détermination de la valeur cinétique inclut la détermination d'une pression et inclut l'ajustement d'une courbe basée sur l'optimisation à une relation de dispersion qui inclut les données de vitesse d'ondes résolues en fréquence.

3. Méthode selon la revendication 2, dans laquelle la relation de dispersion inclut une géométrie du volume tissulaire.

4. Méthode selon la revendication 3, dans laquelle la géométrie est au moins l'un parmi une sphère et un cylindre.

5. Méthode selon la revendication 4, dans laquelle un volume interne non déformé de l'au moins un parmi une sphère et un cylindre est déterminé à partir d'une épaisseur de paroi de la paroi tissulaire du volume tissulaire.

6. Méthode selon la revendication 1, comprenant en outre la détermination de vitesses de particules axiales dans la paroi tissulaire à l'aide d'une technique d'autocorrélation basée sur une phase.

7. Méthode selon la revendication 6, dans laquelle la détermination de données de vitesse d'ondes résolues en fréquence inclut la réalisation d'une transformation de Fourier 2D des vitesses de particules axiales déterminées.

8. Méthode selon la revendication 6, comprenant en outre la détermination de rapports signal/bruit (SNR) pour des données de vitesses de particules axiales et le rejet de données de vitesses de particules axiales avec des SNR inférieurs à un rapport signal/bruit (SNR) seuil déterminé.

9. Méthode selon la revendication 1, dans laquelle le volume tissulaire est une vessie, et la paroi tissulaire est une paroi vésicale.

10. Méthode selon la revendication 9, comprenant en outre l'ajustement d'une relation de dispersion pour une courbure de la paroi vésicale.

11. Système de détermination d'une valeur cinétique d'un volume tissulaire, le système comprenant :

    un transducteur configuré pour générer des ondes de Lamb (602) dans une paroi tissulaire du volume tissulaire à l'aide d'une force de rayonnement et détecter une énergie ultrasonique réfléchie par plusieurs emplacements le long du volume tissulaire qui est soumis à la force de rayonnement pour former des données d'écho ultrasonique, dans lequel le volume tissulaire est formé par la paroi tissulaire qui sépare spatialement un matériau fluide d'un matériau rigide ;
    un système informatique configuré pour :

       i) déterminer des données de vitesse d'ondes résolues en fréquence à partir des ondes de Lamb générées à partir des données d'écho ultrasonique ; (562) et
       ii) **caractérisé en ce que** le système informatique est en outre configuré pour déterminer une valeur cinétique du volume tissulaire directement à partir des données de vitesse d'ondes résolues en fréquence déterminées. (518)

**12.** Système selon la revendication 11, dans lequel la valeur cinétique est une pression et le système informatique est en outre configuré pour déterminer la pression à l'aide d'un ajustement de courbe basé sur l'optimisation d'une relation de dispersion qui inclut les données de vitesse d'ondes résolues en fréquence.

**13.** Système selon la revendication 12, dans lequel la relation de dispersion inclut une géométrie du volume tissulaire.

**14.** Système selon la revendication 13, dans lequel la géométrie est au moins l'un parmi une sphère et un cylindre.

**15.** Système selon la revendication 14, dans lequel le système informatique est en outre configuré pour déterminer un volume interne non déformé de l'au moins un parmi une sphère et un cylindre à partir d'une épaisseur de paroi de la paroi tissulaire du volume tissulaire.

FIG. 1

TRANSMITTER

CHANNEL #1 PULSE CODE
CHANNEL #2 PULSE CODE
CHANNEL #3 PULSE CODE
CHANNEL #N-1 PULSE CODE
CHANNEL #N PULSE CODE

TRANSMIT CHANNEL CONTROL

900
902
904
906
908

FIG. 2

FIG. 3

START

FORM ACOUSTIC
RADIATION FORCE
EXCITATION SIGNAL — 502

500

APPLY VIBRATION
PULSES TO TISSUE OF
INTEREST — 504

ACUIRE ULTRASOUND
IMAGING FRAMES — 520

DETERMINE MOTION
SIGNAL — 506

SEGMENT IMAGE — 522

PERFORM FOURIER
DOMAIN ANALYSIS OF
TISSUE MOTION VS TIME — 508

DETERMINE
GEOMETRY AND
COMPARTMENT WALL
THICKNESS — 524

CALCULATE PHASE
VELOCITY DISPERSION — 510

DETERMINE LARGE-
STRAIN DEFORMATION
OF COMPARTMENT — 526

516 — NEXT
VIBRATION
FREQUENCY

FIT MEASURED PHASE VELOCITY
DISPERSION TO PRE-STRESSED
ANTI-SYMMETRIC LAMB-WAVE
MODEL — 512

N — LAST
FREQUENCY
? — 514

Y

618 — DETERMINE
KINETIC
VALUE — 518

END

FIG. 4A

FIG. 4B

600

Ultrasound
Probe

602

Ultrasound
System

Transverse Wave

630

608

Data
Processing
for Kinetic
Value
Estimation

601

Pressurized Biological
Compartment

FIG. 5A

Waves
induced by
focused
ARFI pulse

Imaging of
displacement
from waves

Imaging of
compartment
thickness and
geometry

605

630

608

606

630

608

606

608

FIG. 5B

FIG. 6A

FIG. 6B

FIG. 6C

FIG. 6D

FIG. 7

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63215912

- US 2014296709 A1

**Non-patent literature cited in the description**

- Ultrasound bladder vibrometry method for measuring viscoelasticity of the bladder wall. **IVAN Z NENADIC et al.** PHYSICS IN MEDICINE AND BIOLOGY. INSTITUTE OF PHYSICS PUBLISHING, 03 April 2013, vol. 58, 2675-2695